# EUROPEAN PATENT SPECIFICATION

(11) **EP 2 042 103 B1**
(45) Date of publication and mention of the grant of the patent: **02.03.2011**
(21) Application number: 08253113.8
(22) Date of filing: 24.09.2008
(51) Int. Cl.: A61B 17/02, A61B 17/30

(54) **Inflatable medical device**
Aufblasbare medizinische Vorrichtung
Dispositif médical gonflable

(30) Priority: 25.09.2007 US 860570
(43) Date of publication of application: 01.04.2009
(73) Proprietor: Ethicon Endo-Surgery, Inc., Cincinnati, OH 45242 (US)
(72) Inventor: Kathrani, Biten Kishore, Mumbai 400 052 Maharashtra (IN); Gadgil, Ulhas Sadashiv, Thane 400 606 Maharashtra (IN); Jain, Hitesh, Chittorgarh 312 001 Rajasthan (IN)
(74) Representative: Tunstall, Christopher Stephen

(56) References cited:
- WO-A-2005/104959
- NL-C2- 1 029 010
- US-A- 6 042 539
- US-A1- 2004 097 792

## Description

### FIELD OF THE INVENTION

The present invention relates generally to a medical device for a tissue-lift or for creating an operative space, and more particularly to a medical device useful in performing vacuum-based minimally invasive procedures or surgeries without the use of insufflation gas.

### BACKGROUND OF INVENTION

Minimally invasive procedures or surgeries (MIP or MIS), including endoscopic, laparoscopic, endoscopically-assisted, or laparoscopically-assisted procedures, are known and offer benefits to a patient such as limited incisional trauma, decreased pain, limited scars, decreased hospitalization, and earlier return to a normal functional state. Other surgical procedures such as Natural Orifice Trans-Luminal Endoscopic Surgeries (NOTES) may offer other benefits such as no incisional trauma, no scarring, and faster recovery. While performing such procedures, it is advantageous to create an operative space between a tissue surface and internal organs, or expand the operative space in the body cavities such as the abdominal or thoracic cavity for improved visualization and better accessibility. The creation or expansion of the operative space typically involves lifting the tissue surface.

Several techniques and devices have been employed to accomplish lifting of the tissue surface or creation of operative space such as gas insufflation, mechanical lifting, and lifting with the help of inflatable bladder or balloon. US 2004/0097792 discusses an apparatus, on which the preamble of claim 1 is based comprising an expansible cage means capable of being inserted into the body through a smell incision or puncture in a collapsed state and an expansion means to selectively expand the cage means inside the body. There can be drawbacks or side effects, however, associated with these techniques, such as increased intra-abdominal pressure, post-operative patient discomfort, and reduced or limited space, visibility and access.

To overcome most of the above-mentioned drawbacks, various medical devices are being developed for lifting tissue surfaces, creating body cavities or expanding body cavities. One such medical device establishes the use of vacuum for creating and maintaining the tissue lift. The device is in the form of a shell or the like, particularly useful in performing gasless endoscopic procedures or surgeries. Further, such devices are also being used for treatment of acute-abdominal compartment syndrome, pre-eclampsia of pregnancy, and other disorders.

For example, U.S. Pat. No. 5,893,368 and U.S. Pat. No. 5,938,626, describe a method and apparatus for lowering intra-abdominal pressure by providing abdominal decompression to a patient on a continuous basis for an extended period of time. Relatively low levels of negative pressure (e.g., -20 to -45 mm Hg) are applied to the patient's abdomen, resulting in the abdominal decompression. This apparatus, in a preferred embodiment, utilizes a patient's urinary bladder pressure as a measure of intra-abdominal pressure, and to control intensity and treatment duration.

In U.S. Pat. No. 6,042,539, a vacuum-actuated tissue-lifting device and method for performing a surgical procedure in an operative space of a patient is disclosed. In one aspect, this device comprises a shell, a vacuum port located on the shell, and an air conduit through the shell.

U.S. Pub. No. 20040049127 describes a tissue perforation method and device, which primarily draws skin and underlying tissue onto it and away from vulnerable underlying structures. This device, in a preferred embodiment, includes a housing having a housing pass-through, a penetrator securely and sealably positioned so that the penetrator device passes through the housing pass-through, and a vacuum system comprising a vacuum source securely and sealably attached through the housing for advancing a patient's tissue onto the penetrator device.

The above-mentioned devices, however, have certain drawbacks that need to be overcome. Firstly, most of these devices use rigid shells or housings that are voluminous and include multiple components such as shell, sealing members, etc., leading to packaging, sterilization and transportation problems. Further, most of these devices being used for MIP or MIS provide limited entry sites for surgical instruments such as trocars, and also the location of the entry sites is fixed. In addition, these devices are designed to be used for a very limited set of patients or procedures.

In the light of the above discussion, Applicant's have recognized the desirability of a medical device that overcomes one or more of the limitations of the devices mentioned above, while keeping one or more of their advantages. Hence, the medical device should be easily transportable and the medical device should be usable for different procedures and over a wider set of patients. Further, the device should provide multiple entry sites for MIPs or MISs, wherein the entry sites can be located according to the requirements of a medical practitioner or surgeon.

### SUMMARY OF INVENTION

In one embodiment, the present invention provides a vacuum-assisted surgical device for lifting a tissue layer. The device comprises a shell having a first un-inflated configuration and a second inflated configuration suitable lifting a tissue layer. The shell can include one or more inflatable ribs for providing the inflated configuration. The shell can include two or more non-inflatable, generally planar segments, each pair of adjacent planar segments being spaced apart by an inflatable rib. The shell can also include a seal, such as a sealing rib extending around the edge of the shell and adapted for sealing engagement with the outer surface of the tissue layer.

In the inflated configuration, the inflatable ribs support the generally planar segments to provide an expansion space between the shell and the tissue layer. The shell can include at least one intake port for inflating the ribs, and at least one suction port for providing vacuum to the expansion space. The surgical device can also include at least one conduit extending through the shell, and at least one entry port extending through one or more of the generally planar segments.

An exemplary, surgical method is provided. The method can include the steps of providing a shell having a first un-inflated configuration and a second inflated configuration; positioning the shell in the un-inflated configuration adjacent a tissue layer of a body; and inflating a portion of the shell to provide an expansion space between the tissue layer and the inflated shell.
In one embodiment, the surgical method includes the steps of providing a shell having at least one inflatable component; inflating the at least one inflatable component to provide an expansion space between the tissue layer and the shell; and providing a vacuum to the expansion space to lift the tissue layer toward the shell. The method can also include providing a conduit extending through the shell and the tissue layer to an operative space within the body, and providing fluid through the conduit into the operative space.

### BRIEF DESCRIPTION OF DRAWINGS

FIG. 1 illustrates an embodiment of a medical device of the present invention in cross-section.

FIG. 2 illustrates a perspective view of the medical device of Figure 1.

FIG. 3 illustrates an arrangement of inflatable ribs and planar segments, in accordance with an embodiment of the present invention.

FIG. 4 illustrates the arrangement of the inflatable ribs and the planar segments, in accordance with another embodiment of the present invention.

FIG. 5 illustrates the arrangement of the inflatable ribs and the planar segments, in accordance with yet another embodiment of the present invention.

FIG. 6 illustrates the entry ports and the conduit means for carrying out a MIP using the medical device, in accordance with an embodiment of the present invention.

FIG. 7A illustrates a side view of an entry port in accordance with an embodiment of the present invention.

FIG 7B illustrates a cross-sectional view of the entry port of Figure 7A taken along section lines A-A.

FIG. 8 illustrates a NOTES procedure using an embodiment of the present invention, and in which gas is communicated to the abdominal cavity via a naturally occurring body orifice (the mouth in Figure 8).

### DETAILED DESCRIPTION OF THE PREFERRED EMBODIMENTS

US 6042539, US 20050159711, and US20050159730, are hereby.

Although the present invention will be described in conjunction with some embodiments as depicted in the figures, a person skilled in the art will easily recognize that numerous additional embodiments will be well within the scope of the present invention, wherein the scope is defined by the claims provided. Hence, the detailed description that follows is intended merely to illustrate the present invention, and is not intended to limit the scope of the claimed invention in any way. In this regard, certain definitions for the terms used in the claims are appropriate to ensure that the reader will not think to limit the scope of these terms to the specified preferred embodiments described in this detailed description. These definitions are given by way of example only, without limitation.

The terms "minimally invasive procedure or surgery (MIP or MIS)" or "minimal access procedure (MAP)" mean medical procedures, including without limitation exploratory, diagnostic, therapeutic, surgical, ambulatory or mobile, emergency, and post mortem procedures, either endoscopic or laparoscopic or endosmotically- or laparoscopically-assisted. "Natural Orifice Trans-Luminal Endoscopic Surgeries (NOTES)" procedures means medical procedures that utilizes naturally occurring body orifice for entering into the body. The body orifice including without limitation mouth, anus, vagina, nose, and ear. Further, the procedure may include without limitations, trans-anal, trans-oral, trans-gastric, trans-colon, and trans-vaginal access.

The term "tissue layer" means a pliable single-layered or multilayered sheet of tissue that covers or lines or connects organs or cells of animals, including without limitations, the skin, the subcutaneous layers, the fascia, the mesentery, the peritoneum, the aponeuroses, the muscular layer, and the like.

The term "operative space" means any working space created within the body, such as below or above, any tissue or any organ by relative separation, such as by lifting partially or fully one or more body structures such as tissue layers, organs, vascular structures, bones, and others, relative to another.

The term "fluid" used herein includes gases, liquids, and combinations thereof.

The term "body cavity" means any fluid-filled space inside the body of a human or an animal, including without limitations, abdominal cavity, thoracic cavity, pelvic cavity, cranial cavity, dorsal cavity, coelom, pseudocoel, and the like. Further, the term "body cavity" also includes potential spaces between tissue layers, organs and tissue layers, and the like.

The term "distal" is used to refer to the portion, part, end, or tip of a component or member that is away from a user, while the term "proximal" is used to describe the portion, part, end, or tip of a component or member which is closer to the user.

Once the scope of some of the critical terms has been defined, to get a more complete understanding of the present invention, a detailed description of the various embodiments of the present invention in conjunction with the illustrations, is provided below.

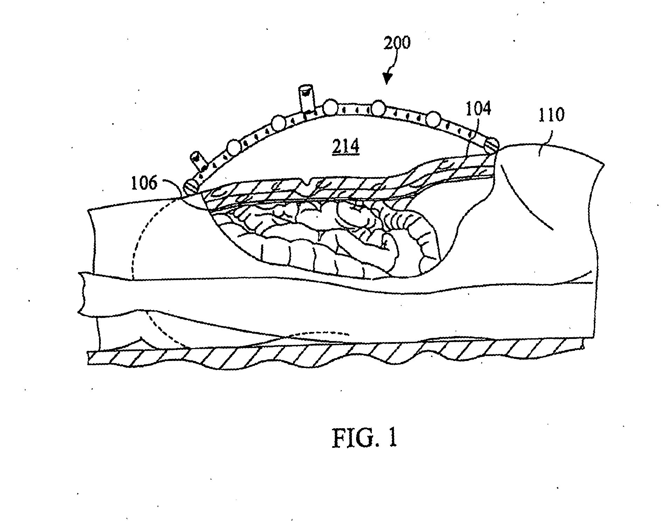

Referring initially to FIG. 1, an illustration of one application of an embodiment of the present is illustrated. Figure 1 illustrates a vacuum-assisted device 200 to lift a tissue layer 104, according to one embodiment of the present invention. The device 200 is shown placed superior to a tissue layer 104 of a patient. In Figure 1, the tissue layer 104 can include the outer layer of the patient's skin. An expansion space 214 is provided between the device 200 and the tissue layer 104. The tissue layer 104 is shown covering an exemplary body cavity 106 of a patient 110, such as the abdominal cavity.

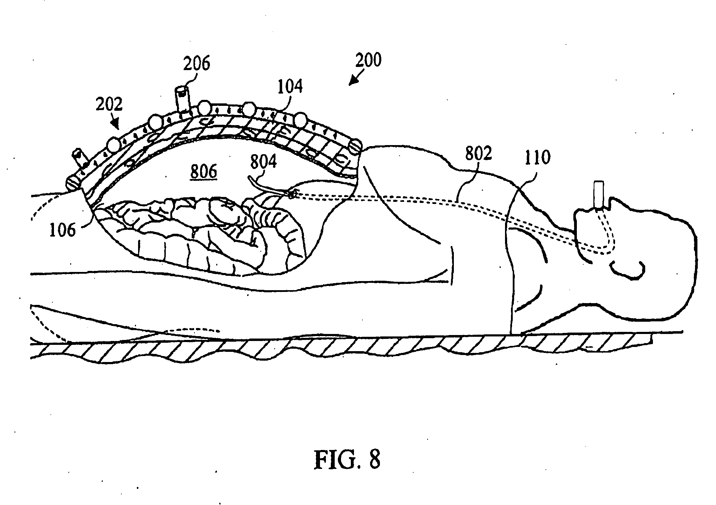

It will be understood to those skilled in the art that body cavity 106 is shown only for illustration purposes. It is possible that the body cavity 106 be replaced by a potential operative space. Further, lifting the tissue layer 104 may create an operative space. In addition, the patient 110 may either be a human or an animal, and the embodiments of the device 200 can be used for both. Further, various embodiments of the device 200 can be useful in performing various procedures such as intra-abdominal decompression, NOTES, open surgical procedures, MIP/MAP, and others. However, the following description illustrates the medical device 200 of being particularly useful in performing vacuum-based gasless (without insufflation gas) MIPs or MAPs (and with reference to Figure 8, a NOTES procedure).

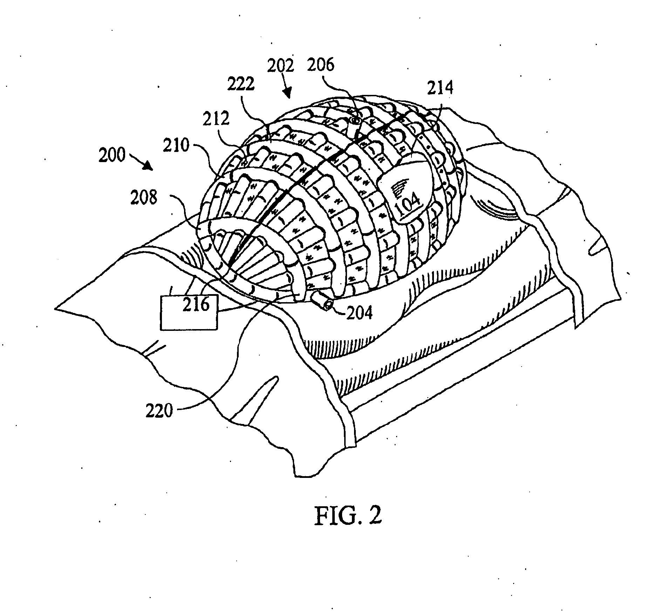

Referring now to FIG.2, one embodiment of the medical device 200 along with its components is illustrated. The medical device 200 illustrated includes a shell 202, an intake port 204, a suction port 206, and a sealing rib 208. In Figure 2, a portion of shell 202 is shown cut away to reveal tissue layer 104 below and an expansion space 214.

The shell 202 further includes a plurality of ribs 210, one or more of which can be inflatable. The shell 202 can also include a plurality of segments 212, which can be generally planar in configuration. The inflatable ribs 210 can be configured to interconnect the or otherwise interlink the planar segments 212 such that, in the inflated condition of the shell 202, the planar segments 212 provide a profile or shape suitable for surrounding the tissue layer 104. When the ribs 210 are inflated, an expansion space 214 is provided between the shell 202 and the tissue layer 104. In the embodiment shown in Figure 2, a generally dome shaped expansion space 214 is provided by the shell 202.

The shell 202 is inflated by supplying a fluid through the fluid supply port 204 coupled to the shell 202. The fluid can be air, water, a disinfected gas, an inert gas, CO2, oil, a gel, or any other suitable fluid. In addition, the intake port 204 can be directly or indirectly in flow communication with all the ribs 210 such that the fluid supplied through the intake port 204 fills and inflates all the inflatable ribs 210.

A fluid supply (not shown) such as a fluid pump including a one-way valve can be employed to provide fluid to intake port 204. Once inflated, the ribs 210 provide sufficient strength and stiffness to the shell 202 to withstand a vacuum applied through the suction port 206.

Further, one or more non-inflatable ribs, such as a non-inflatable rib 216 shown in Figure 2, can be provided for providing additional support and strength to the shell 202. In Figure 2 the rib 216 extends in an arcuate fashion along the major circumferential dimension of the shell 202. The non-inflatable rib 216 can comprise self-organizing metal linkages, a self-organizing spine with one or more tensioning wires, a rib made of one or more shape memory materials (such as Nitinol), or other non-inflatable, flexible ribs (such as ribs formed of metals, plastics, rubber, or composites) which allow the shell 202 to be collapsed or folded, such as for packaging or storage.

The suction port 206 can be coupled to the shell 202 such that the suction port 206 is in communication with the expansion space 214. The suction port 206 can be located at or near the apex of the shell 202. A vacuum source (not shown) can be provided to apply vacuum to the expansion space 214 through the suction port 206. The application of the vacuum to the expansion space 214 results in lifting the tissue layer 104 into the expansion space 214.

A sealing rib 208 can be provided to maintain the vacuum effectively in the expansion space 214. The sealing rib 208 can be positioned around the perimeter of the shell, such as along the rim of the shell 202 by means of adhesive bonding, thermal bonding, or any other suitable bonding or fastening method. The sealing rib 208 conforms to the topology of the external surface of the tissue layer 104 and provides a fluid seal to maintain vacuum established in expansion space 214. The sealing rib 208 can be formed of any suitable flexible material, and can be both flexible and elastically extendible. For instance, the sealing rib 208 can be formed of a material such as silicone, rubber, open or closed cell foam, and the like.

If desired, the sealing rib 208 can include one or more fasteners or sealing features for assisting in releasably sealing the shell 202 against the tissue layer 104. For instance, the sealing rib 208 can include an adhesive layer (such as a pressure sensitive adhesive layer covered by contact release paper) positioned on an inferior surface of the sealing rib 208 so that the sealing rib 208 can be releasably secured to the tissue layer 104 extending along the boundary of the sealing rib 208. It will be, however, understood to those skilled in the art that fastening means is not limited to the adhesive layers and may include other fastening means such as belts, straps, VELCRO™ type fasteners, and the like.

In another embodiment of the present invention, the sealing rib can comprise multiple rib segments arranged parallel to the rim of the shell 202. If desired, the sealing rib 208 (or rib segments) can be inflatable. For instance, the sealing rib 208 can be inflated as needed, to conform to the topology of the external surface of the tissue layer 104. If desired, the sealing rib 208 can be coupled to a fluid supply and a valve (not shown in FIG.2) can be provided to control the amount of inflation of the rib 208. Alternatively, the sealing rib 208 can communicate with a fluid supply via the intake port 204, so that the inflatable ribs 210 and the sealing rib 208 are interconnected and can be inflated together.

The generally planar segments 212 and the inflatable ribs 210 can be separately formed and coupled or connected together by any suitable means, such as adhesive bonding, ultrasonic bonding, thermal bonding, or the like. Alternatively, the segments 212 can be formed integrally (such as by extrusion or other forming techniques) with the ribs 210. If desired, the inflatable ribs 210 can be double walled layers and can be formed of an elastically extensible material. By way of example, ribs 210 can be formed from a material such as silicon, rubber, or a medical grade polymer, such as polymeric film.

The inflatable ribs 210 can be separately inflatable, or can be interconnected to each other to allow the fluid supplied through the intake port 204 to fill and inflate all the ribs 210 together. The inflatable ribs 210 can each be interconnected to the sealing rib 208 along the rim of the shell 202, for example at a plurality of junctions 220, as shown in FIG.2. The inflatable ribs 210 can be interconnected together a plurality of junctions 222 as shown in FIG.2.

The segments 212 and the ribs 210 can be formed of a transparent or semitransparent material that is substantially impermeable to air. In an embodiment of the present invention, the material is flexible, elastically extensible, and can have a thickness penetrable by using one or more surgical instruments such as trocars, incision blades, and the like. For example the material can be a film or sheet formed of medical grade silicone, rubber, polymer, composites, and the like. In one embodiment of the present invention, the planar segments 212 can be segments of a continuous penetrable sheet, film, or membrane attached to the inner surface of the inflatable ribs 210.

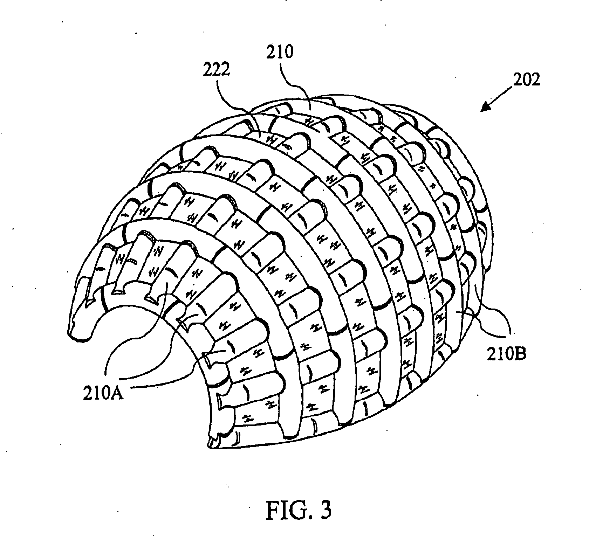

In one embodiment, the inflatable ribs 210 can be interconnected at substantially right angles, and the segments 212 can be generally rectangular in shape as shown in Figure 3. Figure 3 illustrates inflatable ribs 210 as generally tube shaped ribs 210A and 210B terminating at the rim of the shell 202. The inflatable ribs 210A/B extend along sagittal and transverse planes of the patient, and they interconnect or intersect at generally right angles with one another to form substantially rectangular shaped windows. The planar segments 212 occupy the rectangular shaped windows. It will be, however, understood that ribs 212 may be positioned along other planes or directions and can interconnect or intersect at various angles with one another to result in windows of different shapes or designs there between, and not limited to the one shown and described in Figure 3. Correspondingly the planar segments 212 may have different shapes or designs.

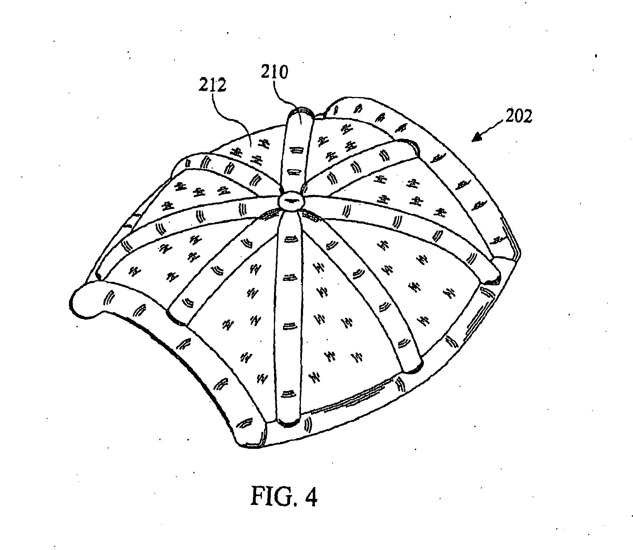

Referring to Figure 4, device 200 can include tube shaped ribs 210 extending in a generally radial manner from the apex of the shell 202 (in the inflated condition) to terminate at the rim of shell 202. The inflatable ribs 210 can be positioned along capital and transverse planes of the patient, and they can meet one another at the apex of shell 202. Hence, the inflatable ribs 210 form substantially triangular shaped windows that are occupied by the planar segments 212.

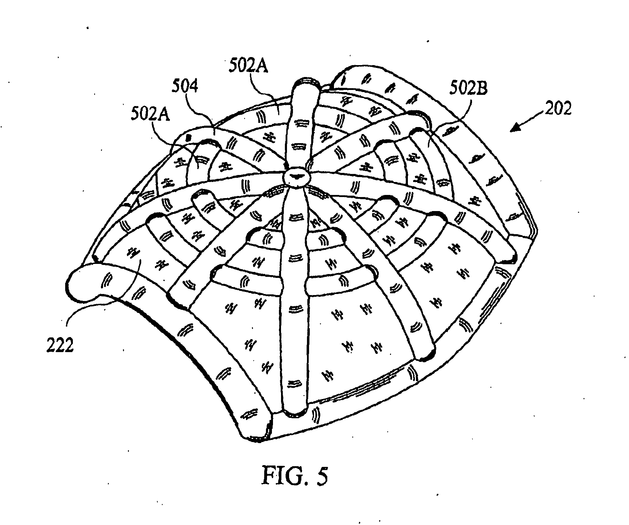

In yet another embodiment of the present invention, as shown in FIG. 5, the device 200 includes a two or more generally ring shaped ribs indicated as 502A and 502B. Rib 502A has a diameter and length less than the diameter and length of rib 502B. The ribs 502A and 502B can be arranged along planes generally parallel to the coronal plane of the patient. The device 200 can include another set of ribs 504 starting at the apex of the shell 202 (in inflated condition) and extending generally radically to terminate at the rim of shell 202. The ribs 504 can be positioned along capital and transverse planes of the patient, and interconnect or intersect at the apex of shell 202. Hence, windows of varying shapes and sizes result, which are occupied by the planar segments 212.

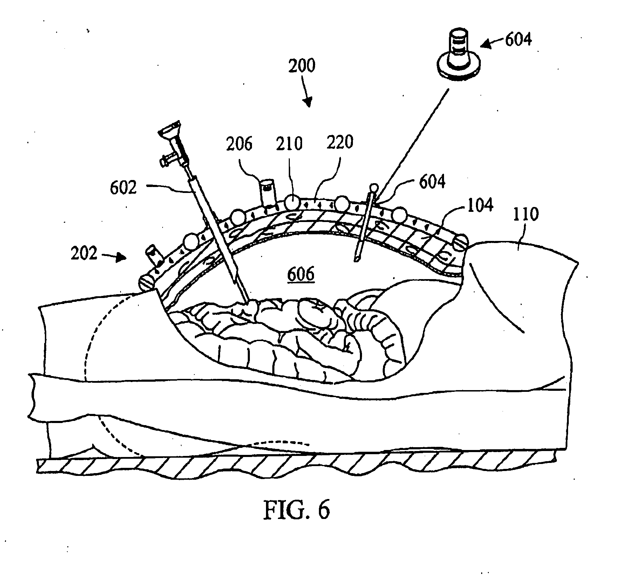

Figure 6 illustrates various other components that can be used for MIP, MIS, or MAP, procedures. For instance, a conduit 602 and entry ports 604 are illustrated, in accordance with an embodiment of the present invention. The entry ports 604 and conduit means 602 are particularly useful for carrying out a MIP using the medical device 200. Before going into the details of these additional components, it should be understood that application of the vacuum to the expansion space 214 leads to lifting of the tissue layer 104. As the tissue layer 104 is lifted, the body cavity 106 of the patient 110 is decompressed. This decompression of the body cavity 106 is particularly useful for treatment of certain conditions, such as (when the body cavity 106 is the abdominal cavity) acute-abdominal compartment syndrome, pre-eclipse of pregnancy, and others. Also, lifting the tissue layer 104 can be beneficial in NOTES procedures to provide improved visualization and larger working space.

Referring to Figure 6, an operative space 606 can be created or expanded inside the body cavity 106. The operative space 606 can be created or enlarged by lifting the tissue layer toward the shell 202. In addition, incisions can be made through the shell 202 and the tissue layer 104. The incisions enable the surgical instruments to access the operative space 606.

Still referring to Figure 6, the operative space 606 inside the body cavity 106 can be created or expanded by providing a vacuum to lift tissue layer 104 while permitting passage of a gas through the conduit 602 into the body cavity 106. The gas enters the body cavity 106 and facilitates enlargement of the operative space 606 as the tissue layer 104 is lifted into the expansion space 214 and towards the shell 202 (such as by application of vacuum through the suction port 206). The passage of gas into the body cavity 106 allows internal tissues or organs of the body to separate from the tissue layer 104. The gas provided through conduit 602 can be any suitable fluid, including without limitation air, a compressed gas, a sterilized gas, CO2, and the like.

The conduit 602 can be coupled to the shell 202 and extends through the shell 202 and the tissue layer 104 into the operative space 606. In an embodiment of the present invention, the conduit 602 is coupled/integrated with one or more of the surgical instruments used for carrying out the MIP. Where the present invention is employed in a NOTES procedure, an air conduit can be provided via an endoscopic access device (e.g. an endoscope) used to accesses the body cavity 106 through the natural orifices (as illustrated in Figure. 8 and described more fully below).

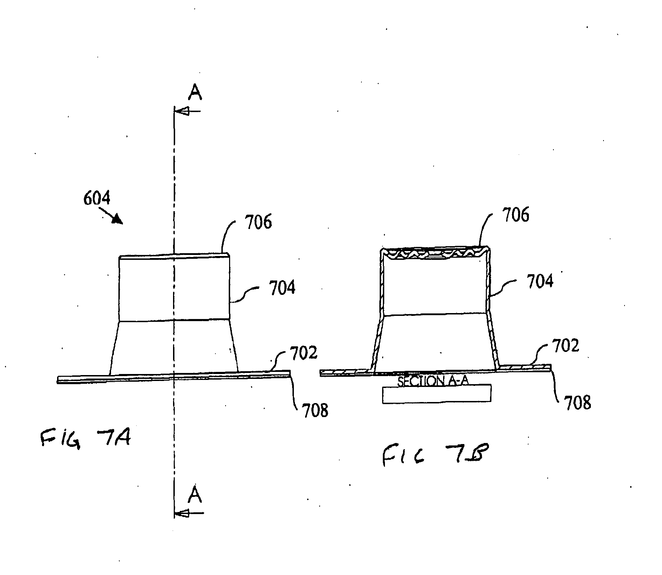

Surgical instrument access to the operative space 606 can be provided via one or more entry ports 604 illustrated in Figures 6 and 7. The penetrable nature of the planar segments 212 allows locating entry ports 604 at various locations on the shell 202 as desired by the surgeon or medical practitioner.

For instance, the surgeon can select the points of incision on the tissue layer 104 during or before the surgery and can select the planar segments 212 to be penetrated, corresponding to the points of incision. The surgeon can affix the entry ports 604 at the sites of penetration on the shell 202. Thereafter, the selected planar segments can be penetrated by one or more access instruments such as a blade knife, a scalpel, a scissor, a cutting tool, and the like. The entry ports 604 provide the surgical instruments access to the operative space 606. The entry ports 604 can be formed of an elastic and flexible material to allow the surgeon or the medical practitioner to orient the surgical instruments as desired within limited span.

Referring to Figures 7A and 7B, each entry port can include a flange 702, a cylindrical wall 704 and an integrated sealing member 706 for maintaining the vacuum in the expansion space 214. The sealing member 706 provides a sliding seal about the instruments extending into the port 604 by allowing the instrument inserted in the port 604 to be advanced and retracted while maintaining vacuum within shell 202. The sealing member 706 can be a flexible disc shaped member. Apart from sealing the entry into the shell 202, the sealing member 706 can also provide support for the surgical instrument inserted therethrough, and hence provide better maneuverability. In various embodiments of the present invention, the sealing member 706 can be a duck bill valve, a iris valve, a bicuspid valve, a tricuspid valve, and the like.

Still referring to Figures 7A and 7B, the entry ports 604 can include an affixing member 708 for affixing the entry ports 604 at the sites of penetration on the shell 202. Suitable affixing members 708 include, but are not necessarily limited to, one or more adhesive layers (such as a pressure sensitive adhesive layer). The entry ports 604 can be provided in various sizes and shapes.

FIG. 8 illustrates an arrangement for carrying out a NOTES procedure using the medical device 200, in accordance with an embodiment of the present invention. In Figure 8, a medical device 200 is shown disposed above the patient 110. An access channel 802 (such as a working channel of a gastroscope) is provided, where the channel 802 extends from a natural orifice (such as mouth of the patient 110 in Figure 8) into the body cavity 106.

In Figure 8, the access channel 802 passes through the stomach (trans gastric) into the abdominal cavity, such as through an incision in the stomach wall. A flexible surgical cannula 804 is also shown extending from the access channel 802 and through the incision in the stomach wall. The cannula 804 passes through the access channel 802 for providing surgical instruments access to the body cavity 106.

In the embodiment shown in Figure 8, the operative space 806 inside the body cavity 106 is created or expanded by permitting passage of a gas through the a conduit associated with the cannula 804, and into the body cavity 106. The gas fills the body cavity 106 as the tissue layer 104 is lifted into the expansion space 214 towards the shell 202 by application of vacuum through the suction port 206. The passage of gas into the body cavity 106 allows internal tissues or organs of the body to separate from the tissue layer 104. Hence, space is created for easy manipulation of the surgical instruments and enhanced visualization in the operative space 806. While Figure 8 illustrates providing gas to body cavity 106 via the mouth, in various other embodiments of the present invention, the natural orifice can be the anus, vagina, ear, nose, and the like. Further, the access channel 802 may pass through the stomach, esophagus, colon, cervix, culd-de-sac, rectum, and other lumens of the body, as needed for the particular procedure being performed.

The various embodiments of the medical device 200 provide improved visualization and adequate operative space for carrying out various procedures, such as MIP/MAP/MIS, or NOTES, or a combination thereof, on the patient in an effective and efficient manner. In addition, because the medical device 200 can be collapsed or folded (such as prior to inflation or after deflation of shell 202), the medical device 200 can be easily transported and/or included in a kit of objects including the shell 202, conduit 602 and ports 604. Further, the shell 202 can be formed as a single integrated unit, and can conform to fit a wide range of patients or procedures. The material used to form the shell 202 can be relatively inexpensive, and the shell 202 can be a single use, disposable unit.

While the present invention has been illustrated by description of several embodiments, it is not the intention of the applicant to restrict or limit the scope of the appended claims to such detail. Numerous other variations, changes, and substitutions will occur to those skilled in the art without departing from the scope of the claims.

## Claims

1. A vacuum-assisted surgical device (200) for lifting a tissue layer (104), the device comprising a shell (202) having a first un-inflated configuration and a second inflated configuration suitable lifting a tissue layer;
wherein the shell comprises:
a plurality of inflatable ribs (210);
a plurality of generally planar segments (212) coupled to the ribs;
wherein the ribs, when inflated, support the planar segments to provide an expansion space (214) between the shell and the tissue layer; and
the device further comprises at least one intake port (204) in communication with at least one of the inflatable ribs;
**characterised in that** the device further comprises at least one suction port (206) for providing suction to the expansion space (214).

2. The surgical device of Claim 1 further comprising at least one non-inflatable rib (216).

3. The surgical device of Claim 1 further comprising at least one conduit (602) providing a passageway through the shell.

4. The surgical device of Claim 1 wherein each generally planar segment is formed of a generally transparent material.

5. The surgical device of Claim 1 wherein each generally planar segment is adapted to be penetrated by a piercing instrument.

6. The surgical device of Claim 1 further comprising at least one entry port (604) for providing surgical instrument access to an operative space (606) located below the tissue layer.

7. The surgical device of Claim 6, wherein the entry port comprises an integrated sealing member (706) to maintain the vacuum of the operative space while providing access to the surgical instrument.

8. The surgical device of Claim 1 further comprising a seal (208) disposed along an edge of the shell for providing sealing between the shell and a tissue layer.

## Patentansprüche

1. Vakuumunterstützte chirurgische Vorrichtung (200) zum Anheben einer Gewebeschicht (104), wobei die Vorrichtung eine Schale (202) umfasst, die eine erste nicht aufgeblasene Konfiguration und eine zweite aufgeblasene Konfiguration zum Anheben einer Gewebeschicht aufweist;
wobei die Schale umfasst:
eine Vielzahl von aufblasbaren Rippen (210);
eine Vielzahl von allgemein planaren Segmenten (212), die mit den Rippen gekoppelt sind;
wobei die Rippen im aufgeblasenen Zustand die planaren Segmente tragen, um einen Expansionsraum (214) zwischen der Hülle und der Gewebeschicht bereitzustellen; und
die Vorrichtung ferner mindestens einen Einlassstutzen (204) in Verbindung mit mindestens einer der aufblasbaren Rippen umfasst;
**dadurch gekennzeichnet, dass** die Vorrichtung ferner mindestens einen Absaugstutzen (206) zum Bereitstellen einer Absaugung für den Expansionsraum (214) umfasst.

2. Chirurgische Vorrichtung nach Anspruch 1, ferner umfassend mindestens eine nicht aufblasbare Rippe (216).

3. Chirurgische Vorrichtung nach Anspruch 1, ferner umfassend mindestens eine Rohrleitung (602) zum Liefern eines Durchgangs durch die Schale.

4. Chirurgische Vorrichtung nach Anspruch 1, **dadurch gekennzeichnet, dass** jedes allgemein planare Segment aus einem allgemein transparenten Material gebildet ist.

5. Chirurgische Vorrichtung nach Anspruch 1, **dadurch gekennzeichnet, dass** jedes allgemein planare Segment gestaltet ist, um von einem Durchstechinstrument durchdrungen zu werden.

6. Chirurgische Vorrichtung nach Anspruch 1, ferner umfassend mindestens einen Zugangsstutzen (604) zum Liefern eines Zugangs für ein chirurgisches Instrument zu einem Operationsraum (606), der sich unter der Gewebeschicht befindet.

7. Chirurgische Vorrichtung nach Anspruch 6, **dadurch gekennzeichnet, dass** der Zugangsstutzen ein integriertes Abdichtungselement (706) zum Aufrechterhalten des Vakuums des Operationsraumes unter gleichzeitiger Bereitstellung eines Zugangs für das chirurgische Instrument umfasst.

8. Chirurgische Vorrichtung nach Anspruch 1, ferner umfassend eine Abdichtung (208), die entlang eines Randes der Schale angeordnet ist, um eine Abdichtung zwischen der Schale und einer Gewebeschicht bereitzustellen.

## Revendications

1. Dispositif chirurgical assisté sous vide (200) pour soulever une couche de tissu (104), le dispositif comprenant une coque (202) ayant une première configuration non gonflée et une seconde configuration gonflée adaptée pour soulever une couche de tissu ;
dans lequel la coque comprend :
une pluralité de nervures gonflables (210) ;
une pluralité de segments généralement plans (212) raccordés aux nervures ;
dans lequel les nervures, quand elles sont gonflées, supportent les segments plans pour fournir un espace de dilatation (214) entre la coque et la couche de tissu ; et
le dispositif comprend en outre au moins un orifice d'entrée (204) en communication avec au moins une des nervures gonflables ;
**caractérisé en ce que** le dispositif comprend en outre au moins un orifice d'aspiration (206) pour fournir une aspiration à l'espace de dilatation (214) .

2. Dispositif chirurgical selon la revendication 1, comprenant en outre au moins une nervure non-gonflable (216) .

3. Dispositif chirurgical selon la revendication 1, comprenant en outre au moins un conduit (602) fournissant un passage à travers la coque.

4. Dispositif chirurgical selon la revendication 1, dans lequel chaque segment généralement plan est formé d'un matériau généralement transparent.

5. Dispositif chirurgical selon la revendication 1, dans lequel chaque segment généralement plan est adapté pour être pénétré par un instrument de perçage.

6. Dispositif chirurgical selon la revendication 1, comprenant en outre au moins un orifice d'entrée (604) pour fournir un accès pour l'instrument chirurgical à un espace opérationnel (606) situé sous la couche de tissu.

7. Dispositif chirurgical selon la revendication 6, dans lequel l'orifice d'entrée comprend un élément d'étanchéité intégré (706) pour maintenir le vide de l'espace opérationnel tout en fournissant un accès pour l'instrument chirurgical.

8. Dispositif chirurgical selon la revendication 1, comprenant en outre un joint (208) disposé le long d'un bord de la coque, afin de procurer une étanchéité entre la coque et une couche de tissu.
